Europäisches Patentamt

European Patent Office

Office européen des brevets

(19)

(11) **EP 0 685 220 B1**

(12) **FASCICULE DE BREVET EUROPEEN**

(45) Date de publication et mention
de la délivrance du brevet:
**30.08.2000 Bulletin 2000/35**

(51) Int Cl.$^7$: **A61K 7/135**, A45D 19/00

(21) Numéro de dépôt: **95401032.8**

(22) Date de dépôt: **04.05.1995**

(54) **Procédé de décoloration des cheveux par irradiation avec un laser, et dispositif**

Verfahren zum Bleichen von Haaren durch Bestrahlung mit einem Laser, sowie die Vorrichtung

Process for hair bleaching by laser irradiation, and this apparatus

(84) Etats contractants désignés:
**AT BE CH DE ES FR GB IT LI NL SE**

(30) Priorité: **04.05.1994 FR 9405469**

(43) Date de publication de la demande:
**06.12.1995 Bulletin 1995/49**

(73) Titulaire: **L'OREAL, S.A.**
**75008 Paris (FR)**

(72) Inventeurs:
• **Caisey, Laurence**
**F-94400 Vitry-sur-Seine (FR)**

• **Monnais, Christian**
**F-92200 Neuilly-sur-Seine (FR)**
• **Samain, Henri**
**F-91570 Bievres (FR)**
• **Sturla, Jean-Michel**
**F-92210 Saint-Cloud (FR)**

(74) Mandataire: **Tonnellier, Jean-Claude**
**Nony & Associés,**
**29, rue Cambacérès**
**75008 Paris (FR)**

(56) Documents cités:
**WO-A-91/06279** **US-A- 4 792 341**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

L'invention concerne la décoloration des cheveux. Plus précisément, l'invention a pour objet un procédé de décoloration des cheveux par irradiation avec un rayonnement laser, ainsi qu'un dispositif pour mettre en oeuvre ce procédé.

On sait que pour décolorer ou éclaircir les cheveux, on utilise classiquement un traitement chimique à l'aide d'un agent oxydant, tel que le peroxyde d'hydrogène ou des persels, qui détruit au moins une partie des produits colorants naturels et/ou artificiels présents dans les cheveux.

La méthode classique de décoloration chimique des cheveux nécessite l'emploi d'agents oxydants relativement puissants et/ou concentrés, qui ont pour effet de dégrader non seulement les substances colorantes, mais aussi la fibre kératinique des cheveux. Il en résulte que les cheveux ainsi décolorés sont fragiles et doivent ensuite être traités avec précaution.

Pour ces raisons, il est impossible d'appliquer à de tels cheveux divers traitements cosmétiques tels que par exemple le traitement de déformation permanente utilisé pour les cheveux naturels, qui consiste à appliquer sur les cheveux un agent réducteur, à des pH relativement élevés, puis un agent oxydant, et ces agents ont des effets nettement agressifs sur la fibre kératinique des cheveux. En fait, l'application d'un traitement classique de déformation permanente sur une chevelure décolorée par voie chimique provoque des dégradations irréversibles et même des cassures des cheveux.

Par ailleurs, on sait qu'une proportion importante de personnes souhaite obtenir une chevelure dite "méchée", c'est-à-dire une chevelure qui n'est pas totalement décolorée, mais dans laquelle seulement certaines mèches de cheveux sont décolorées. Il s'agit d'une opération dite de "méchage". Le cas des chevelures méchées est particulièrement délicat, car on retrouve alors sur une même chevelure des cheveux naturels et des cheveux très décolorés.

La présente invention permet de remédier à ces divers inconvénients, grâce à un procédé de décoloration des cheveux par irradiation à l'aide d'un faisceau laser, dans des conditions particulières qui permettent d'obtenir, sur tous types de cheveux, colorés naturellement ou artificiellement, une décoloration d'intensité facilement contrôlable, sans dégradation notable de la fibre kératinique, et avec une durée de traitement raisonnable. Ce procédé est particulièrement bien adapté à l'obtention d'une chevelure méchée. En outre, les cheveux ainsi décolorés, conservent les propriétés mécaniques et physico-chimiques qu'ils avaient avant la décoloration. Ils peuvent par exemple être soumis sans délai à un traitement de permanente, avec les compositions de permanente classiques.

La possibilité théorique de décolorer des cheveux avec un rayonnement laser a été mentionnée dans la publication Tech. News, *Laser Focus*, Vol. 19, No. 9, p.26, Septembre 1983. En outre, dans le brevet US-4 792 341, on a décrit un dispositif expérimental permettant d'étudier la destruction de la mélanine des cheveux à l'aide d'un rayonnement laser. En fait, ce brevet US ne décrit ni un procédé ni un dispositif permettant, en pratique, l'application de l'irradiation laser à la décoloration des cheveux.

On a maintenant découvert qu'il est possible de décolorer des mèches de cheveux, quelle que soit leur couleur d'origine (naturelle ou artificielle), sans dégradation de la fibre kératinique, en soumettant successivement des parties des mèches de cheveux à traiter à une irradiation par un faisceau laser à condition de choisir une puissance du rayonnement laser adaptée au type de cheveux à décolorer.

Des études sur cheveux isolés de diverses origines (cheveux naturels européens, japonais, mexicains et scandinaves) ont permis d'étudier la puissance lumineuse nécessaire pour obtenir une bonne décoloration des cheveux sans dégradation de la fibre kératinique, avec une seule impulsion laser (tir monocoup). On a constaté que les cheveux très foncés (japonais et mexicains) ne se décolorent pas suffisamment en profondeur ou éclatent sous l'irradiation lorsqu'on augmente encore la puissance par unité de surface, pour une durée d'impulsion donnée. En utilisant une puissance crête inférieure à la puissance crête la plus élevée qui, en tir monocoup, pour la durée d'impulsion utilisée et pour le type de cheveux étudié, ne provoque pas l'éclatement de la fibre kératinique, on a ensuite effectué des tirs successifs, en séquences, sur une même zone des cheveux isolés traités. On a découvert que les cheveux isolés, même les cheveux très foncés du type japonais ou mexicain, pouvaient être décolorés sans dommage en abaissant ainsi la puissance crête et en effectuant plusieurs passages successifs sur une zone traitée, ce qui permet de décolorer d'abord les couches superficielles, puis les couches plus profondes et finalement les cheveux peuvent être décolorés totalement.

Des études analogues faites sur des mèches de cheveux ont permis de constater qu'il est possible de décolorer les cheveux disposés en mèches, à condition de sélectionner une puissance du rayonnement laser adaptée au type de cheveux traités. Cette puissance doit être suffisante pour dégrader ou détruire la mélanine, mais ne doit pas dépasser un certain seuil. On a découvert que plus la couleur naturelle des cheveux est foncée, plus ce seuil doit être faible. On arrive donc à ce résultat surprenant que plus les cheveux sont difficiles à décolorer, plus la puissance du rayonnement laser doit être modérée.

Comme cela apparaîtra évident pour les spécialistes, c'est en fait l'énergie fournie aux grains de mélanine, sur une durée de temps suffisamment courte, qui doit être suffisante pour dégrader ou détruire la mélanine. C'est donc en réalité la densité d'énergie fournie par unité de surface, en un temps suffisamment court, qui doit atteindre un seuil suffisamment élevé pour que les cheveux puissent être décolorés. Dans la présente demande, lorsqu'on parle de "puissance" ou de "puissance crête", il faut comprendre qu'il s'agit d'une simplification de langage, car c'est en fait l'énergie fournie lors de chaque impulsion qui est importante, et il faut donc tenir compte de la durée de l'impulsion, qui doit être toutefois non supérieure à une microseconde environ (durée approximative du temps de relaxation de la mélanine) dans le cas de la décoloration des cheveux naturels.

On a également découvert qu'il est possible de décolorer de façon analogue les cheveux colorés artificiellement, à condition d'opérer, comme pour les cheveux non teints, en adaptant la puissance du rayonnement laser à la couleur naturelle des cheveux. Ce n'est qu'après avoir effectué cette étape préliminaire, correspondant à la dégradation de la mélanine, que l'on peut entreprendre la décoloration proprement dite, correspondant à la dégradation du colorant artificiel. En effet, la dégradation des colorants artificiels nécessite des énergies plus importantes que la dégradation de la mélanine, de sorte que si l'on cherchait à dégrader directement lesdits colorants, les cheveux seraient détruits par éclatement de la fibre kératinique, comme dans les expériences rapportées ci-dessus.

On sait par ailleurs que certaines personnes âgées ont une chevelure "blanche" qui a en fait une teinte jaunâtre peu esthétique. Le procédé de l'invention permet de transformer cette coloration jaunâtre en blanc pur.

L'invention a donc pour objet un procédé de traitement de décoloration des cheveux, tel que défini dans la revendication 1.

On sait qu'un laser se compose essentiellement d'un milieu actif rendu amplificateur par un mécanisme de pompage fournissant de l'énergie aux atomes de façon sélective, ledit milieu actif étant enfermé dans une cavité résonnante. Le milieu actif est alors capable d'émettre un faisceau lumineux, sensiblement monochromatique, polarisé et cohérent. En raison de cette cohérence, un faisceau laser concentre une énergie nettement plus importante que celle d'un rayonnement émis par une source lumineuse classique.

Certains lasers, notamment ceux à milieu actif solide, sont capables d'émettre un rayonnement laser sous la forme d'impulsions très brèves (généralement entre la femtoseconde et la microseconde). La concentration de l'énergie sur des intervalles de temps aussi brefs confère à l'impulsion laser une puissance, dite puissance crête, considérable. On utilise de préférence dans le procédé de l'invention des lasers permettant la production d'impulsions contrôlées. On peut utiliser par exemple des lasers à rubis ou des lasers dont le milieu actif contient des ions de terres rares ou d'actinides, par exemple un laser à néodyme. La réalisation de tels lasers est bien connue. Les ions actifs peuvent être insérés dans une matrice cristalline telle que le grenat d'yttrium-aluminium (en abrégé YAG), ou dans une matrice amorphe telle qu'un verre. On règle la fréquence de récurrence des impulsions à l'aide d'une lampe de pompage à éclairs. On peut régler l'énergie disponible à l'aide de systèmes classiques, notamment des polariseurs.

On utilise de préférence des lasers émettant dans l'ultraviolet proche, dans le visible ou dans le proche infrarouge, par exemple à des longueurs d'onde de 300 à 1100 nm. On peut utiliser par exemple un laser YAG-néodyme qui émet à 1,06 $\mu$m éventuellement avec un multiplicateur de fréquence qui permet par exemple d'obtenir une émission de longueur d'ondes 532 nm (fréquence double) ou de 355 nm (fréquence triple).

La détermination du couple puissance crête-durée d'impulsion (par exemple la puissance crête du faisceau laser pour une durée d'impulsion donnée) peut être aisément déterminée en tenant compte des conditions concernant la densité d'énergie par impulsion, en fonction de la couleur naturelle des cheveux, même s'il s'agit de cheveux teints. Plus précisément, il faut veiller à ne pas dépasser une densité d'énergie maximum par impulsion qui est de l'ordre de :

- 0,35 J/cm$^2$ pour les cheveux brun foncé (de type japonais ou mexicain),
- 0,4 J/cm$^2$ pour les cheveux châtain foncé,
- 0,5 J/cm$^2$ pour les cheveux châtain clair,
- 0,7 J/cm$^2$ pour les cheveux blond foncé,
- 1,2 J/cm$^2$ pour les cheveux blond clair.

Les diverses données fournies ci-dessus concernant la densité d'énergie ont été établies pour un rayonnement de longueur d'onde 532 nm. Lorsque la longueur d'onde utilisée est différente, il convient d'appliquer un facteur de correction

$$\frac{\lambda \text{ nm}}{532}$$

comme cela est exposé plus en détail dans la partie expérimentale ci-après.

Par ailleurs, on rappelle que les différentes teintes de cheveux peuvent être définies de façon objective par la luminance (L), selon le système de coordonnées colorimétriques CIE-(L,a,b). Dans la présente demande, les teintes des cheveux mentionnées correspondent aux gammes de luminance mentionnées ci-après :

EP 0 685 220 B1

| Type de cheveux | L |
|---|---|
| japonais ou mexicain | inférieure à 18 |
| châtain foncé | 18-20 |
| châtain clair | 22-24 |
| blond foncé | 28-35 |
| blond clair | 45-52 |

Le procédé décrit ci-dessus, qui correspond à une décoloration au moins partielle par dégradation de la mélanine des cheveux, doit être effectué dans tous les cas, même si l'on traite les cheveux teints à l'aide d'un agent colorant, et dans ce dernier cas, le procédé de décoloration comporte une étape supplémentaire d'irradiation des cheveux, analogue à celle décrite ci-dessus, mais par un rayonnement laser fournissant une densité d'énergie plus élevée, suffisante pour détruire ou dégrader l'agent colorant ; cette densité d'énergie est notamment au moins égale à 0,8 J/cm$^2$, par impulsion, à 532 nm, et est généralement inférieure à 2 J/cm$^2$.

Pour mettre en oeuvre l'étape préliminaire de dégradation de la mélanine, il suffit de connaître la couleur naturelle des cheveux teints à traiter, et on applique alors des conditions d'irradiation convenant pour les cheveux ayant cette couleur naturelle, lesdites conditions ayant été déterminées préalablement, une fois pour toutes, par de simples expériences de routine.

La durée des impulsions peut aller par exemple de 10 picosecondes à 100 nanosecondes.

Le procédé de l'invention nécessite évidemment de décolorer la chevelure par mèches, en irradiant successivement des zones de ladite mèche. Généralement, la surface d'irradiation peut varier dans la gamme de 0,1-2 cm$^2$.

Le procédé de l'invention est donc particulièrement bien adapté à l'obtention de chevelures méchées. Pour traiter successivement les zones à décolorer par déplacement relatif d'une mèche par rapport au faisceau laser, on peut déplacer la mèche par rapport à l'appareil utilisé pour le traitement, ou vice-versa, et/ou faire varier périodiquement la direction du faisceau laser de façon à effectuer des balayages successifs de la zone traitée. Cette variation périodique de la direction du faisceau laser peut être obtenue par exemple à l'aide d'un miroir oscillant.

On a constaté que lorsqu'on effectue la décoloration par des tirs laser en séquence, les cheveux avaient tendance à s'échauffer localement. Pour que cet échauffement local ne soit pas dommageable pour les cheveux, il convient de limiter la fréquence de récurrence des impulsions, et/ou de procéder à des déplacements relatifs suffisants de la mèche de cheveux traitée par rapport au faisceau laser de façon à éviter des échauffements cumulés, après quoi on peut revenir sur une zone précédemment traitée, ayant eu le temps de refroidir suffisamment, pour compléter le traitement, et ainsi de suite. En pratique, il est facile de déterminer une fréquence convenable de récurrence des impulsions et/ou une vitesse appropriée de déplacement de la mèche de cheveux par rapport au faisceau laser, par de simples expériences de routine, ladite fréquence convenable et/ou ladite vitesse appropriée étant celles pour lesquelles on n'observe pas d'échauffement dommageable des cheveux. On peut choisir par exemple une fréquence de récurrence des impulsions pouvant aller de 5 à 50 Hz, en particulier de 10 à 20 Hz.

Les cheveux traités peuvent être secs ou humides.

Pour pouvoir décolorer la mèche de cheveux traitée dans de bonnes conditions, il est préférable de disposer cette mèche sous la forme d'une nappe de cheveux, et on peut alors décolorer les cheveux de la zone traitée en irradiant au moins une des faces de ladite nappe. De préférence, l'épaisseur de la nappe de cheveux correspond à l'épaisseur de 3 à 20 cheveux superposés environ.

Selon un mode de réalisation particulier, le procédé de l'invention est caractérisé par le fait :

- que l'on opère à l'aide d'un dispositif de traitement comprenant un réceptacle pour la mèche de cheveux à traiter, la surface dudit réceptacle comportant un orifice pour la sortie dudit faisceau laser,
- que l'on applique, sur au moins une partie de leur longueur, les cheveux de ladite mèche étalés en nappe sur la surface dudit réceptacle, de façon qu'une zone à traiter soit en regard dudit orifice,
- que l'on procède à l'irradiation de ladite zone,
- et que par déplacements relatifs de ladite mèche par rapport au faisceau laser, on procède successivement à l'irradiation des autres zones à traiter. On décrira ci-après des dispositifs permettant une telle mise en oeuvre. Avec ces dispositifs, les déplacements relatifs de la mèche par rapport au faisceau laser peuvent être obtenus par des déplacements relatifs de la mèche par rapport audit réceptacle et/ou par variations périodiques de la direction du faisceau laser, comme indiqué précédemment.

L'un des avantages du procédé de décoloration des cheveux par irradiation avec un faisceau laser est qu'il est possible de contrôler en continu la décoloration produite et d'arrêter le traitement au degré de décoloration choisi.

Comme indiqué ci-dessus, l'avantage principal de la décoloration par irradiation laser est que les cheveux ne sont pas dégradés et conservent les propriétés mécaniques et physico-chimiques qu'ils avaient avant traitement. Il s'agit là d'un avantage considérable puisque l'on peut effectuer sur les cheveux ainsi décolorés tous autres traitements cosmétiques sans prendre de précautions particulières.

L'invention a également pour objet un dispositif pour la mise en oeuvre d'un procédé de décoloration des cheveux tel que défini précédemment. Ce dispositif est caractérisé par le fait qu'il comprend

- un corps (1) muni d'un réceptacle (2), ledit réceptacle étant destiné à servir de logement, sur au moins une partie de la longueur des cheveux, pour une mèche de cheveux à traiter étalée en nappe,
- et des moyens (3, 3b, 4, 3a) destinés à acheminer un faisceau laser de façon à irradier au moins une zone d'une mèche de cheveux disposée dans ledit réceptacle.

Dans des modes de réalisation particuliers, le dispositif de l'invention peut encore présenter les caractéristiques suivantes, prises isolément ou, le cas échéant, en combinaison :

- ledit réceptacle comprend une rainure à fond large (2), permettant d'étaler ladite nappe sur ledit fond ;
- ladite rainure peut être associée à une pièce dégageable (5) de forme coopérante capable d'insertion dans ladite rainure en ménageant, entre ladite pièce et le fond de la rainure, un espace constituant un logement pour ladite nappe ; ladite pièce est par exemple dégageable par actionnement d'un levier (7) permettant d'introduire la mèche à traiter dans le réceptacle ou de l'en retirer ; le fond de ladite rainure et/ou une face de ladite pièce en regard dudit fond peut comporter un orifice pour la sortie d'un faisceau laser vers la zone à irradier ;
- dans un autre mode de réalisation, ledit réceptacle comprend une rainure en forme de fente fine débouchant à la surface dudit corps et ayant une profondeur suffisante pour permettre l'insertion de ladite nappe sur la totalité de sa largeur ; de façon analogue à celle mentionnée ci-dessus pour le premier mode de réalisation, l'une au moins des faces en regard de ladite fente fine peut comporter un orifice pour la sortie d'un faisceau laser.

Bien entendu, le réceptacle doit constituer un espace confiné étanche à la lumière pour éviter que le rayonnement laser puisse provoquer des dommages à l'utilisateur ou à sa clientèle.

On va maintenant illustrer l'invention en faisant référence aux dessins annexés dans lesquels :

- la figure 1 est une vue schématique d'un premier mode de réalisation du dispositif de l'invention, qui comprend à son extrémité un réceptacle sous forme de rainure à fond large et une pièce coopérante actionnable par un levier,
- la figure 2 est une vue partielle de dessus de l'extrémité, portant le réceptacle, du dispositif de la figure 1,
- la figure 3 illustre schématiquement le mode d'utilisation du dispositif de la figure 1,
- la figure 4 représente une vue schématique en coupe d'un second mode de réalisation du dispositif, avec un réceptacle en forme de fente fine,
- la figure 5 est une vue en coupe longitudinale verticale du dispositif de la figure 4,
- et la figure 6 schématise le mode d'utilisation du dispositif de la figure 4.

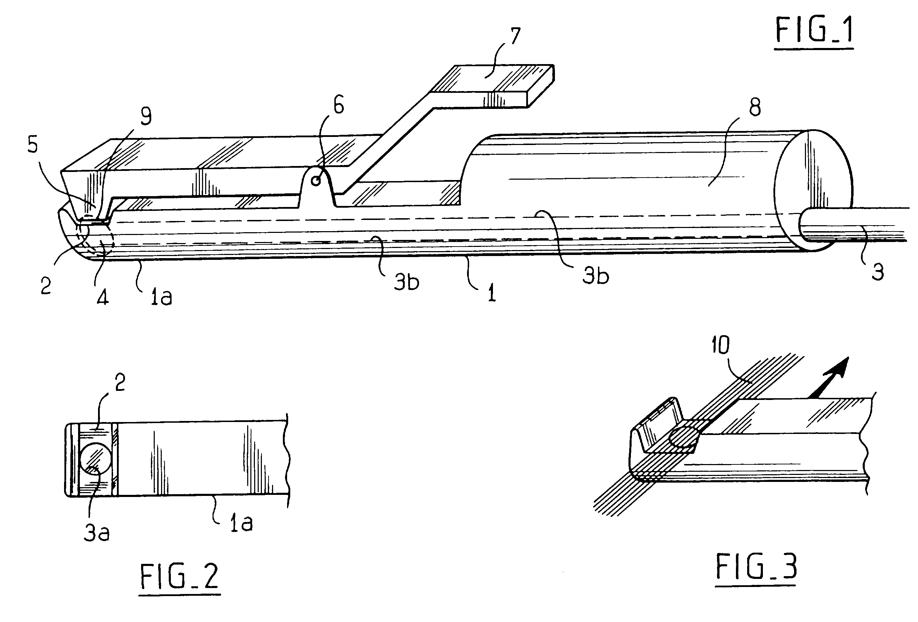

On voit sur la figure 1 que le dispositif comprend un corps allongé (1) partiellement creux dont l'extrémité antérieure (1a) de section réduite, comporte un réceptacle ayant la forme d'une rainure à fond plat (2). Un conduit (3) pour le faisceau laser débouche dans le fond de la rainure (2) après renvoi coudé à l'aide du miroir (4), et forme une lucarne de sortie (3a) à la surface de la paroi (2). Les pointillés (3b) schématisent la propagation du faisceau laser à l'intérieur du corps (1) ou peuvent représenter un guide d'onde.

Une pièce coopérante (5) mobile autour de l'axe (6) est maintenue engagée dans le réceptacle à l'aide de moyens de rappel tels qu'un ressort à tension (non représenté) et est dégageable par actionnement du levier (7) dont est munie la pièce (5) à son extrémité arrière. La partie arrière (8) du corps (1) sert de poignée de prise en main de l'appareil par le manipulateur. Pour décolorer les cheveux, on dégage la pièce (5) pour pouvoir disposer la mèche de cheveux (10), répartie en nappe comme indiqué à la figure 3, puis on laisse la pièce (5) se réengager dans la rainure. La mèche se trouve donc disposée dans l'espace entre les faces (2) et (9), on actionne l'émetteur laser (non représenté) et on déplace le dispositif par rapport à la mèche (ou vice-versa), dans la direction indiquée par la flèche sur la figure 3, pour décolorer successivement toute la mèche ou une partie de mèche à traiter. Il est préférable d'effectuer des déplacements qui ne soient pas trop lents, par exemple de l'ordre de 0,1-5 centimètres par seconde, et en opérant par passages successifs, de traiter à plusieurs reprises les zones à décolorer, ce qui permet de contrôler très facilement l'évolution de la décoloration, dans de bonnes conditions de sécurité. Mais il est également possible d'utiliser des vitesses de déplacement plus faibles, ou des déplacements discontinus, par exemple en liaison avec une fréquence d'impulsions suffisamment basse.

Le corps du dispositif peut être réalisé en tout matériau approprié, par exemple en métal léger comme l'aluminium ou en matière plastique.

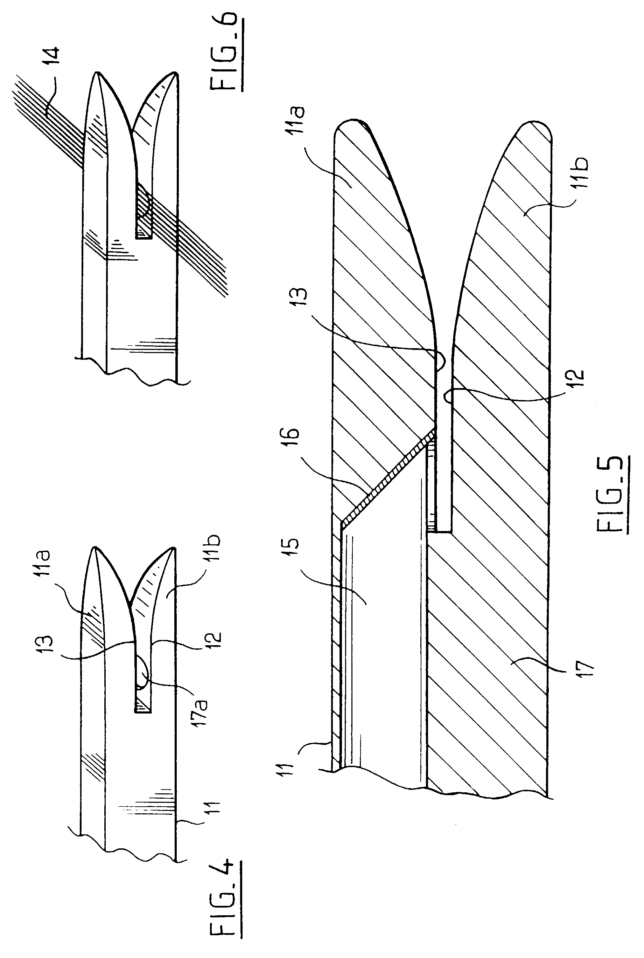

Le dispositif représenté aux figures 4 et 5 comporte un corps allongé (11) dont on n'a représenté que la partie antérieure, la partie arrière non représentée formant poignée. On voit que l'extrémité du corps (11) se sépare en deux branches (11a) et (11b) séparées par une fente dont les faces en regard (12) et (13) constituent un logement pour une mèche de cheveux (14) disposée comme indiqué à la figure 6. Comme dans le premier mode de réalisation, un canal laser (15) débouche sur la face (13) de la fente après renvoi par le miroir (16).

On utilise ce dispositif de façon analogue au précédent.

Les commandes du faisceau laser peuvent être installés sur le dispositif ou dans un organe de commande séparé qui peut être éventuellement actionné au pied. On peut également prévoir des dispositif d'asservissement de la puissance laser, en fonction de la couleur de la mèche à traiter, cet asservissement pouvant être réalisé de façon automatique après lecture de la couleur par un détecteur approprié.

Les exemples suivants illustrent l'invention.

**EXEMPLE 1 :**

Dans cet exemple et dans les exemples suivants, on utilise des mèches de cheveux de 0,25 g, ayant une longueur de 20 cm.

L'appareil utilisé est un appareil du type représenté à la figure 1.

La source de rayonnement laser est un laser Surelite Continuum ; longueur d'onde : 1 Hz 532 nm ; fréquence des tirs ; diamètre du faisceau : 5 mm ; durée de l'impulsion ; 4 ns.

Avec cet appareillage, on a étudié les plages de décoloration optimale, selon la couleur des cheveux à traiter.

Les gammes d'énergie par $cm^2$ pour une impulsion, sont celles qui sont utilisables pour décolorer effectivement les cheveux.

En-dessous de la valeur minimum, il n'y a pas de décoloration notable. Au-dessus de la valeur maximum, la fibre du cheveu éclate ou est fissurée (les dommages sont visibles, selon leur importance, à la loupe binoculaire, au microscope ou au microscope électronique).

Les résultats sont résumés dans le tableau (I) suivant :

TABLEAU (I)

| Cheveux | énergie/$cm^2$ pour 1 impulsion (en J/$cm^2$) |
|---|---|
| japonais | 0,2 à 0,35 |
| châtain foncé | 0,2 à 0,4 |
| châtain clair | 0,15 à 0,5 |
| blond foncé | 0,15 à 0,7 |
| blond clair | 0,1 à 1,2 |

Par ailleurs, l'absorption de l'énergie lumineuse de la mélanine varie avec la longueur d'onde : elle diminue lorsque la longueur d'onde augmente, de sorte que les cheveux supportent sans dégradation une densité d'énergie incidente plus élevée lorsque la longueur d'onde augmente. L'étude expérimentale a montré que la densité d'énergie maximale supportable par les cheveux sans éclatement de la fibre kératinique, pour un rayonnement de longueur d'onde $\lambda$, est sensiblement celle indiquée dans le tableau ci-dessus, multipliée par un facteur

$$\frac{\lambda}{532}$$

où $\lambda$ est exprimée en nanomètres. Cette loi de variation avec la longueur d'onde vaut également pour la relation entre la densité d'énergie incidente et l'efficacité de la décoloration : la densité d'énergie capable de décolorer les cheveux d'un type donné, pour la longueur d'onde $\lambda$, est sensiblement égale à la densité d'énergie permettant d'obtenir une décoloration analogue avec un rayonnement de longueur d'onde 532 nm, multipliée par ledit facteur

$$\frac{\lambda}{532}$$

Pour des cheveux colorés artificiellement, il faut utiliser des densités d'énergie assez élevées, généralement au moins égales à 0,8 J/$cm^2$ par impulsion. Si les cheveux ont été colorés sans décoloration préalable, il faut tenir compte de leur teinte naturelle. Par exemple, si la teinte naturelle des cheveux était châtain clair, il faut d'abord utiliser une densité d'énergie non supérieure à 0,5 J/cm$^2$ (voir tableau 1 ci-dessus) pour décolorer la mélanine. Ce n'est qu'ensuite qu'on pourra utiliser une densité d'énergie supérieure (1 J/cm$^2$ ou plus) pour détruire le colorant artificiel. Si l'on appliquait dès le début cette densité d'énergie élevée, les cheveux éclateraient.

**EXEMPLE 2 :**

On opère comme précédemment, avec un laser BMI présentant les caractéristiques suivantes : longueur d'onde : 523 nm ; fréquence des tirs 10 Hz; diamètre du faisceau : 3 mm; durée de l'impulsion : 30 ps.

La puissance crête par unité de surface correspondant à une décoloration optimale pour des cheveux châtain foncé est de l'ordre de 9,5 GW/cm$^2$, soit une énergie par unité de surface de 0,28 J/cm$^2$.

En outre, on a constaté que la solubilité alcaline et la teneur en acide cystéique des cheveux décolorés par irradiation laser est pratiquement la même que celle des cheveux avant décoloration, alors qu'elles sont fortement augmentées dans le cas d'une décoloration par voie chimique.

**Revendications**

1.  Procédé de traitement de décoloration au moins partielle d'au moins une mèche ou partie de mèche de cheveux, par irradiation de ladite mèche ou partie de mèche à l'aide d'un rayonnement laser de puissance suffisante pour décolorer les cheveux, caractérisé par le fait :

    -   que l'on traite une zone de ladite partie de mèche par irradiation à l'aide d'un faisceau laser émis sous forme d'impulsions pour décolorer au moins partiellement les cheveux de ladite zone, par dégradation de la mélanine des cheveux,
    -   que, le cas échéant, par déplacement relatif de ladite mèche par rapport audit faisceau laser, on traite successivement, de façon analogue, une ou plusieurs autres zones de façon à traiter la totalité de ladite partie de mèche,
    -   que l'on répète éventuellement les traitements précédents jusqu'à obtention du degré de décoloration souhaité pour ladite mèche ou partie de mèche,
    -   que l'on opère avec un rayonnement laser de puissance suffisante pour fournir, par impulsion, une densité d'énergie de 0,1 à 1,2 J/cm$^2$, les valeurs de densité d'énergie étant données ici pour un rayonnement de longueur d'onde de 532 nm, et devant être multipliées par un facteur de correction égal à :

    $$\frac{\lambda}{532}$$

    lorsque le rayonnement utilisé a une longueur d'onde $\lambda$ (exprimée en nanomètres) autre que 532 nm,
    -   et que ladite densité d'énergie choisie n'est pas supérieure à un seuil au-delà duquel la fibre kératinique des cheveux est endommagée, ledit seuil étant d'autant plus faible que la couleur naturelle des cheveux à traiter est plus foncée.

2.  Procédé selon la revendication 1, caractérisé par le fait que l'on opère de façon à ne pas dépasser une densité d'énergie maximum par impulsion qui est de l'ordre de :

    -   0,35 J/cm$^2$ pour les cheveux brun foncé,
    -   0,4 J/cm$^2$ pour les cheveux châtain foncé,
    -   0,5 J/cm$^2$ pour les cheveux châtain clair,
    -   0,7 J/cm$^2$ pour les cheveux blond foncé,
    -   1,2 J/cm$^2$ pour les cheveux blond clair,

    les valeurs de densité d'énergie étant données ici pour un rayonnement de longueur d'onde de 532 nm, et devant être multipliées par un facteur de correction égal à :

    $$\frac{\lambda}{532}$$

    lorsque le rayonnement utilisé a une longueur d'onde $\lambda$ (exprimée en nanomètres) autre que 532 nm..

**3.** Procédé selon la revendication 2, caractérisé par le fait que l'on opère de façon à obtenir une densité d'énergie (en joules/cm$^2$), par impulsion, de :

- 0,2-0,35 pour les cheveux de type japonais,
- 0,2-0,4 pour les cheveux châtain foncé,
- 0,15-0,5 pour les cheveux châtain clair,
- 0,15-0,7 pour les cheveux blond foncé,
- 0,1-1,2 pour les cheveux blond clair,

les valeurs de densité d'énergie étant données ici pour un rayonnement de longueur d'onde de 532 nm, et devant être multipliées par un facteur de correction égal à :

$$\frac{\lambda}{532}$$

lorsque le rayonnement utilisé a une longueur d'onde $\lambda$ (exprimée en nanomètres) autre que 532 nm.

**4.** Procédé selon l'une quelconque des revendications précédentes, caractérisé par le fait qu'en outre, lorsque les cheveux à traiter sont teints à l'aide d'un agent colorant, on effectue une étape supplémentaire d'irradiation des cheveux, de façon analogue à celle décrite dans l'une quelconque des revendications précédentes, mais avec une densité d'énergie plus élevée, suffisante pour détruire ou dégrader l'agent colorant.

**5.** Procédé selon la revendication 4, caractérisé par le fait que ladite densité d'énergie plus élevée est au moins égale à 0,8 J/cm$^2$ par impulsion à 532 nm.

**6.** Procédé selon l'une quelconque des revendications précédentes, caractérisé par le fait que pour éviter un échauffement local dommageable pour les cheveux, on limite la fréquence des impulsions et/ou on procède à des déplacements relatifs suffisants de la mèche à traiter par rapport au faisceau laser.

**7.** Procédé selon l'une quelconque des revendications précédentes, caractérisé par le fait que l'on dispose préalablement ladite mèche de façon à former une nappe de cheveux, et que l'on décolore les cheveux de la zone traitée en irradiant au moins une des faces de ladite nappe.

**8.** Procédé selon l'une quelconque des revendications précédentes, caractérisé par le fait:

- que l'on opère à l'aide d'un dispositif de traitement comprenant un réceptacle pour la mèche de cheveux à traiter, la surface dudit réceptacle comportant un orifice pour la sortie dudit faisceau laser,
- que l'on applique, sur au moins une partie de leur longueur, les cheveux de ladite mèche étalés en nappe sur la surface dudit réceptacle, de façon qu'une zone à traiter soit en regard dudit orifice,
- que l'on procède à l'irradiation de ladite zone,
- et que par déplacements relatifs de ladite mèche par rapport au faisceau laser, on procède successivement à l'irradiation des autres zones à traiter.

**9.** Procédé selon la revendication 8, caractérisé par le fait que lesdits déplacements relatifs comprennent des déplacements relatifs de ladite mèche par rapport audit réceptacle.

**10.** Procédé selon l'une quelconque des revendications 6 et 7, caractérisé par le fait que lesdits déplacements relatifs sont obtenus en faisant varier la direction du faisceau laser de façon à effectuer des balayages de la zone à traiter.

**11.** Dispositif pour la mise en oeuvre d'un procédé tel que défini dans l'une quelconque des revendications précédentes, caractérisé par le fait qu'il comprend:

- un corps (1) muni d'un réceptacle (2), ledit réceptacle étant destiné à servir de logement, sur au moins une partie de la longueur des cheveux, pour une mèche de cheveux à traiter étalée en nappe,
- et des moyens (3, 3b, 4, 3a) destinés à acheminer un faisceau laser de façon à irradier au moins une zone d'une mèche de cheveux disposée dans ledit réceptacle.

**12.** Dispositif selon la revendication 11, caractérisé par le fait que ledit réceptacle comprend une rainure à fond large (2), permettant d'étaler ladite nappe sur ledit fond.

13. Dispositif selon la revendication 12, caractérisé par le fait qu'à ladite rainure est associée une pièce dégageable (5) de forme coopérante capable d'insertion dans ladite rainure en ménageant, entre ladite pièce et le fond de la rainure, un espace constituant un logement pour ladite nappe.

14. Dispositif selon la revendication 13, caractérisé par le fait que ladite pièce est dégageable par actionnement d'un levier (7) permettant d'introduire la mèche à traiter dans le réceptacle ou de l'en retirer.

15. Dispositif selon l'une quelconque des revendications 12 à 14, caractérisé par le fait que le fond de ladite rainure et/ou une face de ladite pièce en regard dudit fond comporte un orifice pour la sortie d'un faisceau laser vers la zone à irradier.

16. Dispositif selon la revendication 11, caractérisé par le fait que ledit réceptacle comprend une rainure en forme de fente fine débouchant à la surface dudit corps et ayant une profondeur suffisante pour permettre l'insertion de ladite nappe sur la totalité de sa largeur.

17. Dispositif selon la revendication 16 , caractérisé par le fait que l'une au moins des faces en regard (12,13) de ladite fente fine comporte un orifice pour la sortie d'un faisceau laser.

**Patentansprüche**

1. Verfahren zur Behandlung zur wenigstens teilweisen Entfärbung von zumindest einer Haarsträhne oder einem Teil einer Haarsträhne durch Bestrahlung der Haarsträhne oder des Teiles der Haarsträhne mit Hilfe einer Laserausstrahlung mit einer ausreichenden Leistung, um die Haare zu entfärben, **gekennzeichnet** dadurch:

    - dass man eine Zone des Teiles der Haarsträhne durch Bestrahlung mit Hilfe eines Laserstrahles in der Form von Pulsen zur Entfärbung oder wenigstens teilweise der Haare der besagten Zone zum Ausbleichen des Melanins der Haare behandelt,
    - dass gegebenenfalls durch Versetzung bzw. Verschiebung der Haarsträhne relativ in Bezug auf den Laserstrahl aufeinander folgend in einer analogen Weise eine oder mehrere andere Zonen in der Art behandelt werden, um die Gesamtheit des Teils der Haarsträhne zu behandeln,
    - dass man eventuell die vorangehenden Behandlungen bis zur Erreichung des gewünschten Ausbleichungsgrades für die Haarsträhne oder den Teil der Haarsträhne wiederholt,
    - dass man mit einer Laserausstrahlung mit einer ausreichenden Leistung zur Versorgung mittels Pulsen mit einer Energiedichte von 0,1 bis 1,2 J/cm$^2$ vorgeht, wobei die Werte der Energiedichte hier gegeben sind für eine Ausstrahlung der Wellenlänge von 532 nm und zuvor mit einem allgemeinen Korrekturfaktor zu multiplizieren ist, wie folgt:

$$\frac{\lambda}{532},$$

    wenn die verwendete Ausstrahlung eine Wellenlänge $\lambda$ (ausgedrückt in Nanometern) hat, die nicht 532 nm beträgt,
    - und dass die ausgewählte Energiedichte nicht über einem Schwellenwert ist, oberhalb dessen die Keratinfaser des Haares beschädigt wird, wobei der Schwellenwert umso mehr schwächer ist, wenn die natürliche Farbe des Haares zur Behandlung dunkler ist.

2. Verfahren nach Anspruch 1, dadurch **gekennzeichnet,** dass man es in der Weise durchführt, um nicht über die maximale Energiedichte durch Pulse hinauszugehen, die in der Größenordnung ist von:

    - 0,35 J/cm$^2$ für dunkelbraune bzw. dunkelbrünette Haare,
    - 0,4 J/cm$^2$ für dunkel-dunkelblonde Haare,
    - 0,5 J/cm$^2$ für hell-dunkelblonde Haare,
    - 0,7 J/cm$^2$ für dunklere blonde Haare,
    - 1,2 J/cm$^2$ für hellere hellblonde Haare,

wobei die Energiedichtewerte hier für eine Ausstrahlung der Wellenlänge von 532 nm angegeben sind und zuvor mit einem allgemeinen Korrekturfaktor zu multiplizieren sind, wie folgt:

$$\frac{\lambda}{532},$$

wenn die verwendete Ausstrahlung eine Wellenlänge $\lambda$ (ausgedrückt in Nanometern) hat, die nicht 532 nm beträgt.

**3.** Verfahren nach Anspruch 2, dadurch **gekennzeichnet,** dass man es in der Weise durchführt, um eine Energiedichte (in Joules/cm$^2$) durch Pulse bzw. Pulsung zu erhalten, von:

- 0,2-0,35 für die Haare vom japanischen Typ bzw. vom dunkelbraunen oder -brünetten Typ,
- 0,2-0,4 für dunkel-dunkelblonde Haare,
- 0,15-0,5 für hell-dunkelblonde Haare,
- 0,15-0,7 für dunklere hellblonde Haare,
- 0,1-1,2 für hellere hellblonde Haare,

wobei die Werte der Energiedichte hier für eine Ausstrahlung der Wellenlänge von 532 nm gegeben sind und zuvor mit einem allgemeinen Korrekturfaktor zu multiplizieren sind, wie folgt:

$$\frac{\lambda}{532},$$

wenn die verwendete Ausstrahlung eine Wellenlänge $\lambda$ (ausgedrückt in Nanometern) hat, die nicht 532 nm beträgt.

**4.** Verfahren nach einem der voranstehenden Ansprüche, dadurch **gekennzeichnet,** dass außerdem, wenn die zu behandelnden Haare mit Hilfe eines Färbungsmittels gefärbt sind, man einen zusätzlichen Bestrahlungsschritt für die Haare derart analog zu irgendeinem der voranstehend beschriebenen Ansprüche einsetzt, aber mit einer zusätzlich angehobenen Energiedichte, die ausreicht, um das Färbungsmittel zu zerstören oder auszubleichen.

**5.** Verfahren nach Anspruch 4, dadurch **gekennzeichnet,** dass die Energiedichte, die weiter angehoben ist, zumindest gleich 0,8 J/cm$^2$ pro Impuls bei 532 nm ist.

**6.** Verfahren nach irgendeinem der voranstehenden Ansprüche, dadurch **gekennzeichnet,** dass man zur Vermeidung einer lokalen schädigenden Aufheizung der Haare die Frequenz der Pulse beschränkt und/oder man mit einer ausreichenden Versetzung bzw. Verschiebung der Haarsträhne, die zu behandeln ist, relativ im Hinblick auf den Laserstrahl verfährt.

**7.** Verfahren nach irgendeinem der voranstehenden Ansprüche, dadurch **gekennzeichnet,** dass man die Haarsträhne zuvor in der Art anordnet, um ein Haarflies auszubilden und dass man die Haare der behandelten Zone ausbleicht, indem man eine der Flächen des Flieses bestrahlt.

**8.** Verfahren nach irgendeinem der voranstehenden Ansprüche, dadurch **gekennzeichnet:**

- dass man mit Hilfe einer Behandlungsvorrichtung vorgeht, die eine Aufnahme für die Haarsträhne der zu behandelnden Haare aufweist, wobei die Oberfläche der Aufnahme eines Öffnung für den Ausgang des Laserstrahls aufweist,
- dass man, zumindest auf einen Teil deren Länge, auf die Haare der zu einem Flies auf der Oberfläche der Aufnahme ausgebreiteten Haarsträhne einwirkt, derart, dass die zu behandelnde Zone in Sicht der Öffnung ist,
- dass man mit der Bestrahlung der Zone fortfährt,
- und dass man durch relative Versetzungen der Haarsträhne im Hinblick auf den Laserstrahl aufeinander folgend mit der Bestrahlung anderer zu behandelnder Zonen fortfährt.

**9.** Verfahren nach Anspruch 8, dadurch **gekennzeichnet,** dass die relativen Versetzungen relative Versetzungen der Haarsträhne im Hinblick auf die Aufnahme umfassen.

**10.** Verfahren nach irgendeinem der Ansprüche 6 und 7, dadurch **gekennzeichnet,** dass die relativen Versetzungen erhalten werden, indem die Richtung des Laserstrahles in der Art verändert wird, um eine Abtastung der zu behandelnden Zone zu bewirken.

**11.** Vorrichtung zur Verwirklichung von einem Verfahren, wie es in irgendeinem der voranstehenden Ansprüche festgelegt ist, dadurch **gekennzeichnet,** dass diese aufweist:

- einen mit einer Aufnahme (2) ausgestatteten Körper (1), wobei die Aufnahme dazu bestimmt ist, um zur Beherbergung von zumindest einem Teil der Länge des Haares für eine zu behandelnde Haarsträhne, die zu einem Flies ausgebreitet ist, zu dienen,
- und mit Mitteln (3, 3b, 4, 3a), die dazu bestimmt sind, einen Laserstrahl derart abzugeben, um zumindest eine Zone einer Haarsträhne auszusetzen, die in der Aufnahme angeordnet ist.

**12.** Vorrichtung nach Anspruch 11, dadurch **gekennzeichnet,** dass die Aufnahme eine große Grundrille bzw. einen großen Grundschlitz (2) aufweist, der die Ausbreitung des Flieses auf dem besagten Grund bzw. Boden ermöglicht.

**13.** Vorrichtung nach Anspruch 12, dadurch **gekennzeichnet,** dass die Rille bzw. Nut einem entfernbaren Abschnitt (5) zugeordnet ist, der Art, die dazu in der Lage ist, in die Rille bzw. Nut eine Einführung zu machen, um zwischen dem Abschnitt und dem Boden bzw. Grund der Rille bzw. der Nut einen Raum bzw. Bereich vorsichtig zu behandeln, der eine Unterbringung für das Flies bzw. Gewebe bildet.

**14.** Vorrichtung nach Anspruch 13, dadurch **gekennzeichnet,** dass der Abschnitt durch Betätigung eines Hebels (7) bzw. Schwenkarmes lösbar ist, wobei die Einfügung der zu behandelnden Haarsträhne in die Aufnahme oder deren Herausnahme ermöglicht wird.

**15.** Vorrichtung nach irgendeinem der Ansprüche 12 bis 14, dadurch **gekennzeichnet,** dass der Boden bzw. Grund der Rille bzw. der Nut und/oder eine Oberfläche des Abschnittes im Hinblick auf den Boden bzw. Grund eine Öffnung für den Ausgang des Laserstrahls in Richtung der zu bestrahlenden Zone aufweist.

**16.** Vorrichtung nach Anspruch 11, dadurch **gekennzeichnet**, dass die Aufnahme eine Rille bzw. eine Nut in der Form von einem feinen Schlitz aufweist, der sich zu der Oberfläche des Körpers öffnet und der von einer Tiefe ist, die ausreicht, um die Einführung des Flieses bzw. des Gewebes über dessen gesamte Länge zu ermöglichen.

**17.** Vorrichtung nach Anspruch 16, dadurch **gekennzeichnet,** dass zumindest die eine der Flächen (12, 13) im Hinblick auf den feinen Schlitz bzw. Riss eine Öffnung für den Ausgang des Laserstrahls aufweist.

**Claims**

**1.** Treatment process for at least partial bleaching of at least a lock or portion of a lock of hair, by irradiation of the said lock or portion of a lock using a laser beam of sufficient power to bleach the hair, characterized:

- in that an area of the said portion of lock is treated by irradiation using a laser beam emitted in the form of pulses in order to bleach, at least partially, the hair in the said area, by degradation of the melanin in the hair,
- in that if required, by relative movement of the said lock with respect to the said laser beam, one or more other areas are treated in succession, in a similar way, so as to treat the totality of the said portion of lock,
- in that the above treatments are possibly repeated until the desired degree of bleaching is obtained for the said lock or portion of lock,
- in that the operation is performed with laser radiation of sufficient power to deliver, per pulse, an energy density of 0.1 to 1.2 $J/cm^2$, the values of energy density being given here for radiation of 532 nm wavelength and having to be multiplied by a correction factor equal to:

$$\frac{\lambda}{532}$$

  when the radiation used has a wavelength $\lambda$ (expressed in nanometres) other than 532 nm,
- and in that the said chosen energy density is not greater than a threshold above which the keratinous fibre of the hair is damaged, the said threshold being lower the darker the natural colour of the hair to be treated.

**2.** Process according to Claim 1, characterized in that the operation is performed so as not to exceed a maximum energy density per pulse which is of the order of:

- 0.35 J/cm$^2$ for dark brown hair,
- 0.4 J/cm$^2$ for dark chestnut hair,
- 0.5 J/cm$^2$ for light chestnut hair,
- 0.7 J/cm$^2$ for dark blond hair,
- 1.2 J/cm$^2$ for light blond hair,

the values of energy density being given here for radiation of 532 nm wavelength and having to be multiplied by a correction factor equal to:

$$\frac{\lambda}{532}$$

when the radiation used has a wavelength $\lambda$ (expressed in nanometres) other than 532 nm.

3.  Process according to Claim 2, characterized in that the operation is performed so as to obtain an energy density (in joules/cm$^2$), per pulse, of:

- 0.2 - 0.35 for Japanese-type hair,
- 0.2 - 0.4 for dark chestnut hair,
- 0.15 - 0.5 for light chestnut hair,
- 0.15 - 0.7 for dark blond hair,
- 0.1 - 1.2 for light blond hair,

the values of energy density being given here for radiation of 532 nm wavelength and having to be multiplied by a correction factor equal to:

$$\frac{\lambda}{532}$$

when the radiation used has a wavelength $\lambda$ (expressed in nanometres) other than 532 nm.

4.  Process according to any one of the preceding claims, characterized in that, furthermore, when the hair to be treated is dyed using a colouring agent, an additional step of irradiation of the hair is performed, in a way similar to that described in any one of the preceding claims, but with a higher energy density, sufficient to destroy or degrade the colouring agent.

5.  Process according to Claim 4, characterized in that the said higher energy density is at least equal to 0.8 J/cm$^2$ per pulse at 532 nm.

6.  Process according to any one of the preceding claims, characterized in that, in order to prevent local heating which can damage the hair, the frequency of the pulses is limited and/or sufficient relative movements of the lock to be treated are carried out with respect to the laser beam.

7.  Process according to any one of the preceding claims, characterized in that, the said lock is arranged beforehand so as to form a ribbon of hair and in that the hair in the treated area is bleached by irradiating at least one of the faces of the said ribbon.

8.  Process according to any one of the preceding claims, characterized:

- in that the operation is performed using a treatment device which includes a receptacle for the lock of hair to be treated, the surface of the said receptacle having an orifice for the exit of the said laser beam,
- in that the hair of the said lock, spread out in the form of a ribbon, is applied, over at least part of its length, to the surface of the said receptacle so that an area to be treated is opposite the said orifice,
- in that irradiation of the said area is then carried out,
- and in that, by relative movements of the said lock with respect to the laser beam, irradiation of the other areas to be treated is carried out in succession.

9. Process according to Claim 8, characterized in that the said relative movements include relative movements of the said lock with respect to the said receptacle.

10. Process according to either of Claims 6 and 7, characterized in that the said relative movements are obtained by varying the direction of the laser beam so as to perform scanning of the area to be treated.

11. Device for implementing a process as defined in any one of the preceding claims, characterized in that it comprises:

    - a body (1) provided with a receptacle (2), the said receptacle being intended to serve as a housing, over at least part of the length of the hair, for a lock of hair to be treated, the lock being spread out in the form of a ribbon,
    - and means (3, 3b, 4, 3a) intended for conveying a laser beam so as to irradiate at least one region of a lock of hair placed in the said receptacle.

12. Device according to Claim 11, characterized in that the said receptacle comprises a wide-bottomed groove (2) allowing the said ribbon to be spread out over the said bottom.

13. Device according to Claim 12, characterized in that the said groove is associated with a disengageable piece (5) of cooperating shape capable of being inserted into the said groove, while leaving, between the said piece and the bottom of the groove, a space constituting a housing for the said ribbon.

14. Device according to Claim 13, characterized in that the said piece is disengageable by the action of a lever (7), allowing introduction of the lock to be treated into the receptacle or its removal therefrom.

15. Device according to any one of Claims 12 to 14, characterized in that the bottom of the said groove and/or one face of the said piece facing the said bottom includes an orifice for the output of a laser beam onto the area to be irradiated.

16. Device according to Claim 11, characterized in that the said receptacle comprises a narrow slot-shaped groove emerging at the surface of the said body and having a depth sufficient to allow insertion of the said ribbon over its entire width.

17. Device according to Claim 16, characterized in that at least one of the facing faces (12, 13) of the said narrow slot includes an orifice for the output of a laser beam.

FIG_1

FIG_2

FIG_3

EP 0 685 220 B1

FIG.4

FIG.6

FIG.5

EP 0 685 220 B1